# EUROPÄISCHE PATENTSCHRIFT

(11) **EP 0 508 343 B1**
(45) Veröffentlichungstag und Bekanntmachung des Hinweises auf die Patenterteilung: **10.06.1998**
(21) Anmeldenummer: 92105896.2
(22) Anmeldetag: 06.04.1992
(51) Int. Cl.: C12H 1/00, C12H 1/22

(54) **Verfahren zur kontinuierlichen Reifung von Bier**
Process for the continuous maturation of beer
Procédé pour la maturation de la bière en continu

(30) Priorität: 12.04.1991 DE 4111879; 14.11.1991 DE 4137474
(43) Veröffentlichungstag der Anmeldung: 14.10.1992
(73) Patentinhaber: Schott Glas, 55122 Mainz (DE); CARL-ZEISS-STIFTUNG trading as Schott Glaswerke, 55122 Mainz (DE)
(72) Erfinder: Aivasidis, Alexander, Dr., W-5161 Merzenich (DE); Wandrey, Christian, Prof., W-5170 Jülich (DE); Breitenbücher, Klaus, Dr., W-6506 Nackenheim (DE); Mistler, Manfred, W-6500 Mainz (DE)

(56) Entgegenhaltungen:
- EP-A- 0 274 587
- EP-A- 0 275 144
- US-A- 4 915 959
- CHEMICAL ABSTRACTS, vol. 112, no. 21, 21. Mai 1990, Columbus, Ohio, US; abstract no. 196633g, Y. WANG ET AL. 'Operation of fluidized-bed bioreactor containing immobilized yeast cells and diacetyl levels in green beer.' Seite 560; Spalte 2/
- BIOTECHNOLOGY Bd. 3, Mai 1985, LONDON Seiten 467 - 470 T. ONAKA ET AL. 'Beer brewing with immobilized yeast.'
- EBC CONGRESS 1991 NOTES Mai 1991, LISBON Seiten 5769 - 5776 AIVASIDIS, A. ET AL. 'Continuous fermentation of alcohol-free beer with immobilized yeast in fluidiyed bed reactors.'

## Beschreibung

Die Erfindung bezieht sich auf ein Verfahren zur kontinuierlichen Reifung von Bier durch weitere Umsetzung der im Primärfermentationsschritt behandelten Würze mit immobilisierter Hefe.

Die Reifung von Bier erfolgt üblicherweise durch Ablagerung des Jungbieres bei niedrigen Temperaturen über einige Wochen hinweg, wodurch eine Geschmacksveränderung und Umwandlung von bei der Primärgärung gebildeten Nebenprodukten resultiert.

Eine solche Ablagerung erfordert nicht nur erhebliche Lagerräume und Vorratshaltung, sondern macht auch die Reaktion der Brauerei auf ungewöhnliche Bedarfsschübe schwierig.

Es wurden daher bereits Versuche zur beschleunigten Reifung von Bier unternommen: So ist aus der DE-PS 21 44 754 ein Verfahren bekannt, bei dem innerhalb von 2 - 3 Tagen durch Zusatz von Kräusen eines mittleren Vergärungsgrades ein Abbau von Diacetyl erreicht werden soll, das als wesentliche zu vermindernde Geschmackskomponente angesehen wird.

Yunji Wang beschreibt ein Wirbelschichtverfahren mit an Calciumalginatgel immobilisierter Hefe, bei dem mit einer Verweilzeit im Reaktor von 2,8 Std. mit einer Zirkulationszahl von 5 eine Primärfermentationsperiode von 14 Std. und ein Diacetylgehalt von 0,5 ppm erreicht werden soll (siehe CA 112 (21) 196 633 g). Über die Bierqualität und Lebensdauer der Anlage werden allerdings keine Angaben gemacht.

A. Willet (Biotechnol. Lett. 10, 7 (1988) S. 473-78) beschreibt Laborversuche zur beschleunigten bakteriellen Bierreifung mit an χ-Carrageenan immobilisierten Mikroorganismen wie Bacillus polymyxa u.a. in konischen Flaschen mit Testmedium. Auch hier ist eine angemessene Beurteilung des Erfolges in geschmacklicher Beziehung schwierig.

Von H. Lommi (Brewing & Distilling Internat. 1990, 22 - 23) wird die Bierreifung mit an einem heterogenen Träger aus DEAE-Cellulose, Polystyrol und Titandioxid aufgewachsener Hefe im Festbett mit kontinuierlicher Durchströmung beschrieben.

T. Onaka u.a. berichten in Bio/Technology 3,5 (1985) S. 467 - 70) über ein Brauverfahren in einem Säulenreaktor mit an Na-Alginat immobilisierter Hefe, bei dem zur Nachbehandlung 10 Std. lang unter Rühren belüftet wird unter Erzielung von 0,05 mg/l Gesamt-Diacetyl.

Aus der US-PS 4 915 959 ist schließlich ein Verfahren zur Bierreifung bekannt, bei dem die primär behandelte Würze unter Überdruck durch eine Reaktionssäule mit an DEAE-Cellulose immobilisierter Hefe hindurchgeleitet wird.

J. Cop et al. (EBC-Kongreß 1989) berichten über Fermentationsuntersuchungen mit in Alginat eingeschlossenen Hefezellen, insbesondere unter Berücksichtigung der Aminosäureaufnahme und -umwandlung. Dabei wurden Verbesserungen serungen der Aminosäure-Ausnutzung in Batch-Fermentation beim Übergang vom Schüttbett zum Fließbett- bzw. Wirbelschichtreaktor gefunden. Gezeigt werden auch Ergebnisse, bei denen poröse Glas-Träger verwendet wurden, deren Einsatzart und Bedeutung jedoch nicht diskutiert werden.

Diese unterschiedlichen Verfahren lassen bislang kein allgemein akzeptiertes Bierreifungsverfahren erkennen.

Ziel der Erfindung ist daher ein möglichst langzeitig zu betreibendes Verfahren zur beschleunigten Reifung von Bier, das vor allem auch dem Reinheitsgebot entspricht.

Das zu diesem Zweck entwickelte erfindungsgemäße Verfahren ist dadurch gekennzeichnet, daß man die Umsetzung mit an offenporigem Sinterglas einer Porosität von 40 - 65 % mit Porengrößen von 60 bis 300 pm immobilisierter Hefe bei erhöhtem Druck durchführt.

Dabei kann kontinuierlich im Festbett oder vorzugsweise im Fließbett gearbeitet werden, bei dem Leerrohrgeschwindigkeiten von 20 - 120 m/h, insb. 30 - 100 m/h und spez. bis 80 m/h zur Aufwirbelung der immobilisierten Hefe vorgesehen werden, die auf Glassinterträgern von ≤ 5 mm, insbesondere von 1 - 2 mm Korngröße aufgewachsen verwendet wird.

Im Festbett wird insbesondere auf Raschigringträgern von 8 - 25 mm φ aufgewachsene Hefe verwendet.

Zwar wurde poröses Glas bereits 1977 (Navarro et al., European J. Appl. Microbiol. 4 (1977) 243 - 54) zur Immobilisierung von Hefe in Betracht gezogen (dgl. DE-OS 28 39 580), und auch von der Anmelderin werden in der DE-OS 34 10 650 sowie in der DE-PS 36 39 153 poröse Gläser als Träger für Mikroorganismen beschrieben und auch die Möglichkeit zur Erzeugung von Gärungsprodukten sowie Anwendung im Festbett-oder Wirbelschichtreaktor erwähnt, jedoch hat dieser globale Hinweis offensichtlich nicht richtungsweisend auf die Entwicklung spezieller Biergewinnungsverfahren gewirkt, für die bislang - insbesondere im Rahmen der Bierreifung - Alginate und Carrageenate sowie Cellulosederivate vorgesehen werden.

Gemäß der Erfindung wird als Träger insbesondere offenporiges Kalk-Natron- oder Borosilikatglas bevorzugt. Zweckmäßig sind Reifungstemperaturen von 2 - 15°C und Verweilzeiten von 1 - 15 h, insbesondere von etwa 10 h, wobei als Steuergröße zweckmäßigerweise der im Produkt gewünschte niedrige Diacetylgehalt dient.

An diesem Verfahren sind außer dem inerten (Glas)Träger lediglich die natürlichen Ausgangsstoffe der Bierbrauerei beteiligt und somit das Reinheitsgebot beachtet und apparativ sowie prozeßtechnisch Voraussetzungen für einen langanhaltenden störungsfreien Betrieb mit guter Regelbarkeit gegeben.

Als Hefeträger für das Wirbelschichtverfahren dienen insbesondere weitporige Sinterglasperlen von ≤ 2 mm φ mit einer Porosität um 50 %, die möglichst hoch sein soll, um einen guten Zugriff von Medium und Stoffaustausch an der in den Poren befindlichen Hefe zu ermöglichen, die damit trotz des bei der Sekundärfermentation im Vergleich zur Primärfermentation erheblich geminderten Nährstoffangebots ausreichend aktiv bleibt, so daß die bei der Reifung notwendige Restwürzeumwandlung und Minderung des Zuckergehalts rasch erreicht wird. Gleichzeitig wird der Diacetylgehalt - soweit vorhanden - ausreichend herabgesetzt, wofür insbesondere eine kontinuierliche Überwachung auf Maximalgehalte von ≤ 0,1 mg/l dient, die auf einer On-line Analytik und meßwertabhängigen Steuerung der Prozeßparameter, wie Temperatur und Verweilzeit, basiert.

Die Fermentation erfolgt zweckmäßigerweise bei Drucken von 2 - 6 bar, insbesondere von 2,5 - 3 bar, die sich durch CO₂-Entwicklung aufbauen.

Als Reaktor eignet sich ein Wirbelschichtreaktor mit einer äußeren Umlaufpumpe, die für einen ausreichenden Durchfluß sorgt. Am oberen Ende hat der säulenförmige Reaktor eine Querschnittserweiterung, die für eine ausreichende Verlangsamung der Flüssigkeitsströmung und damit Umkehr des suspendierten Kornmaterials sorgt.

Bei einem solchen Reaktor macht das Wirbelschichtvolumen etwa 50 - 60 % des gesamten Arbeitsvolumens aus, und das Schüttvolumen liegt bei etwa 25 - 30 % dieses Arbeitsvolumens.

Mit einem solchen Wirbelschichtreaktor ist eine befriedigende Steuerung für eine gleichbleibende Qualität des Reaktorauslaufs gewährleistbar.

Die Mikroorganismen werden auf dem Trägermaterial vorzugsweise im Wirbelschichtreaktor in einer Anfahrphase angezüchtet, indem Hefekonzentrat bei 20 -35°C als Inokulat in den Reaktor gegeben wird und mit Verweilzeiten unterhalb der Generationszeit der Hefe dafür gesorgt wird, daß während der Kolonisierungsphase des Trägermaterials ein optimaler Selektionsdruck zugunsten sessiler Mikroorganismen bei einem hohen Level der Raumzeitausbeute stattfinden kann. Zweckmäßigerweise erfolgt eine solche Anfahrphase über eine Woche hinweg und kann durch regelmäßige Probennahme und rasterelektronenmikroskopischen Auswertung überwacht werden. Selbstverständlich sind je nach Bedingungen kürzere Anfahrphasen von 2 - 3 Tagen bzw. auch längere Zeitdauern von bis zu mehreren Wochen möglich.

Das gewählte Trägermaterial von hoher offener Porosität und relativ geringer Teilchengröße ermöglicht die Zurückhaltung hoher Biomassekonzentrationen, welche um ein Mehrfaches über den beim trägerfreien konventionellen Verfahren mit Batch-Betrieb liegen. Die gemessenen Biotrockenmassegehalte liegen bei 25 - 30 g/l Wirbelschictvolumen.

Besonders bevorzugt wird Siran®-Granulat der Firma Schott Glaswerke, Mainz mit einer Porendoppelstruktur mit großen durchgehenden Poren, die einen guten Stoffaustausch begünstigen, und kleinen fixierungsfördernden Mikroporen in den Wänden der Makroporen.

Die angefügte Figur zeigt einen Wirbelschichtreaktor analog zu der Anordnung gemäß DE-Patentschrift 41 11 879 C2, auf die hier ausdrücklich Bezug genommen wird. Der Zulauf des Reaktors stammt aus einem Vorlagetank für primär-vergorene Würze und wird mittels einer Pumpe kontinuierlich entnommen. Die Umlaufpumpe sorgt für die Träger-Fluidisierung im Reaktor durch Einstellung einer Lineargeschwindigkeit von 15 - 50 m/h. Die rezirkulierte Flüssigkeit passiert einen Wärmeaustauscher zur Einstellung der gewünschten Temperatur. Zur Konstanthaltung derselben ist der Wirbelschichtreaktor mit einer äußeren Wärmedämmung versehen.

Mit Hilfe einer konduktometrischen Niveauregelung wird eine Produktpumpe angesteuert, so daß der Reaktor bei konstantem Volumen betrieben wird. Gestrichelt angedeutet ist eine rechnergestützte Prozeßsteuerung und -überwachung mit Hilfe eines Personal-Computers zur Variation der Verweilzeit, um einen gewünschten Diacetylgrenzwert nach On-line Konzentrationserfassung einzustellen.

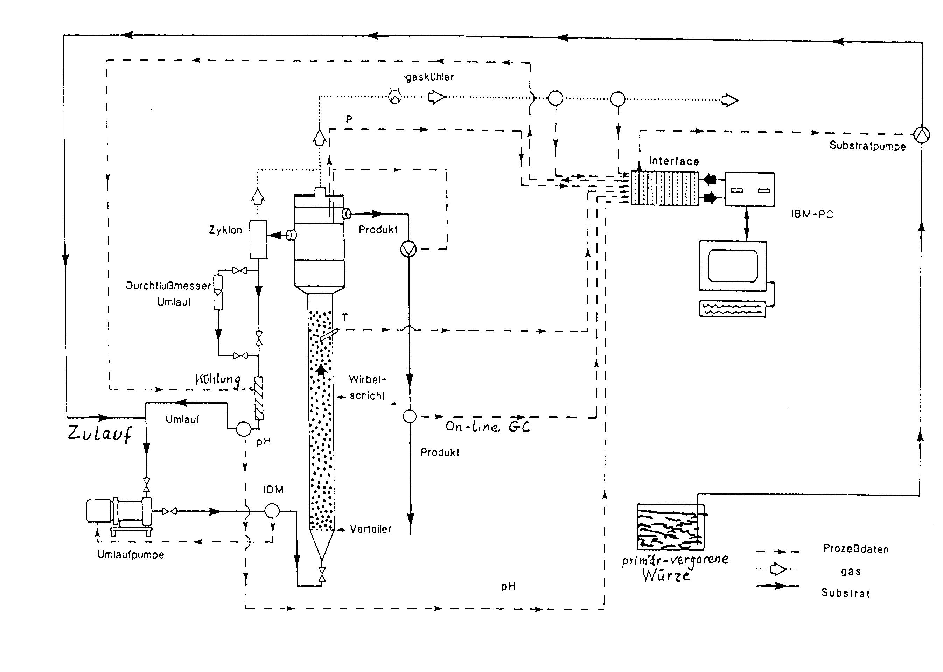

## Patentansprüche

1. Verfahren zur kontinuierlichen Reifung von Bier durch weitere Umsetzung der im Primärfermentationsschritt behandelten Würze mit immobilisierter Hefe,
**dadurch gekennzeichnet,**
daß man die Umsetzung mit an offenporigem Sinterglas einer Porosität von 40 - 65 % mit Porengrößen von 60 bis 300 µm immobilisierter Hefe bei erhöhtem Druck durchführt.

2. Verfahren nach Anspruch 1,
**dadurch gekennzeichnet,**
daß man im Fließbett mit Sinterglasträgern von ≤ 5 mm Korngröße und Leerrohrgeschwindigkeiten von 20 - 120 m/h, insb. 30 - 100 m/h arbeitet.

3. Verfahren nach Anspruch 2,
**dadurch gekennzeichnet,**
daß man Träger mit 1 - 2 mm Teilchengröße verwendet.

4. Verfahren nach Anspruch 1,
**dadurch gekennzeichnet,**
daß man im Festbett mit Raschigring-Trägern von 8 - 25 mm Durchmesser arbeitet.

5. Verfahren nach einem der vorangehenden Ansprüche,
**dadurch gekennzeichnet,**
daß bei Temperaturen von 2 - 15°C und Verweilzeiten von 1 - 15 Std. gearbeitet wird.

6. Verfahren nach einem der vorangehenden Ansprüche,
**dadurch gekennzeichnet,**
daß die Umsetzung bei Drucken von 2 - 6 bar erfolgt.

7. Verfahren nach einem der vorangehenden Ansprüche,
**gekennzeichnet durch**
eine kontinuierliche Überwachung des Diacetylgehalts auf Werte ≤ 0,1 mg/l.

8. Verfahren nach einem der vorangehenden Ansprüche,
**dadurch gekennzeichnet,**
daß man durch Hefekonzentrat-Inokulation bei 20 - 35°C und Verweilzeiten unterhalb der Generationszeit der Hefe über einige Tage hinweg auf dem porösen Träger aufgewachsene Hefe verwendet.

## Claims

1. Process for the continuous maturation of beer by further reaction with immobilized yeast of the wort treated in the primary fermentation step, characterized in that the reaction is carried out at elevated pressure with yeast immobilized on open-pore sintered glass of a porosity of 40 - 65% having pore sizes of 60 to 300 µm.

2. Process according to Claim 1, characterized in that the process is carried out in the fluid bed using sintered glass supports of ≤ 5 mm in particle size and superficial velocities of 20 - 120 m/h, in particular 30 - 100 m/h.

3. Process according to Claim 2, characterized in that supports having a particle size of 1 - 2 mm are used.

4. Process according to Claim 1, characterized in that the process is carried out in a fixed bed using Raschig ring supports of 8 - 25 mm in diameter.

5. Process according to one of the preceding claims, characterized in that the process is carried out at temperatures of 2 - 15°C and residence times of 1 - 15 hours.

6. Process according to one of the preceding claims, characterized in that the reaction is carried out at pressures of 2 - 6 bar.

7. Process according to one of the preceding claims, characterized by continuous monitoring of the diacetyl content to values ≤ 0.1 mg/l.

8. Process according to one of the preceding claims, characterized in that use is made of yeast grown on the porous support over several days by yeast concentrate inoculation at 20 - 35°C and residence times below the generation time of the yeast.

## Revendications

1. Procédé pour la maturation de la bière en continu, par autre réaction supplémentaire du moût traité dans l'étape de fermentation primaire, avec de la levure immobilisée,
caractérisé en ce que l'on réalise la réaction avec une levure immobilisée sur un verre fritté à pores ouverts, d'une porosité de 40 à 65%, avec une taille des pores de 60 à 300 µm, à pression élevée.

2. Procédé suivant la revendication 1,
caractérisé en ce que l'on travaille en lit fluidisé avec des supports de verre fritté de taille des grains ≤ 5 mm et des vitesses de tube vide de 20 à 120 m/heure, en particulier de 30 à 100 m/heure.

3. Procédé suivant la revendication 2,
caractérisé en ce que l'on utilise un support avec une taille des particules de 1 à 2 mm.

4. Procédé suivant la revendication 1,
caractérisé en ce que l'on travaille dans un lit solide avec des supports de type anneaux de Raschig d'un diamètre de 8 à 25 mm.

5. Procédé suivant l'une quelconque des revendications précédentes, caractérisé en ce que l'on travaille à des températures de 2 à 15°C et avec des temps de séjour de 1 à 15 heures.

6. Procédé suivant l'une quelconque des revendications précédentes,
caractérisé en ce que la réaction est réalisée à des pressions de 2 à 6 bars.

7. Procédé suivant l'une quelconque des revendications précédentes,
caractérisé par un contrôle continu de la teneur en diacétyle à des valeurs ≤ 0,1 mg/litre.

8. Procédé suivant l'une quelconque des revendications précédentes,
caractérisé en ce que l'on utilise la levure développée sur le support poreux, par inoculation du concentré de levure à 20-35°C et avec des temps de séjour inférieurs à la période de génération de la levure et durant quelques jours.
